# EUROPÄISCHE PATENTANMELDUNG

(11) **EP 3 141 295 A1**
(43) Veröffentlichungstag der Anmeldung: **15.03.2017**
(21) Anmeldenummer: 15184639.1
(22) Anmeldetag: 10.09.2015
(51) Int. Cl.: B01D 53/22, B01D 53/30, C10L 3/10

(54) **VORRICHTUNG UND VERFAHREN ZUR AUFTRENNUNG EINES GASGEMISCHES MITTELS EINER MEMBRANEINHEIT**

(71) Anmelder: Axiom Angewandte Prozeßtechnik Ges. m.b.H., 2443 Ebreichsdorf (AT)
(72) Erfinder: SZIVACZ, Johannes, 2443 Ebreichsdorf (AT); WINTERSPERGER, Johannes, 2443 Ebreichsdorf (AT)
(74) Vertreter: Sonn & Partner Patentanwälte

(57) **Zusammenfassung**

Die Erfindung betrifft eine Vorrichtung zur Auftrennung eines Gasgemisches in Produktgas und Offgas mittels Gaspermeation mit einer Membraneinheit (1) und einem der Membraneinheit (1) vorgeschalteten, vorzugsweise drehzahlregelbaren Verdichter (3), welche Membraneinheit (1) einen Gaseingang (1a), einen Retentat- bzw. Produktgasausgang (1b) und einen Permeat- bzw. Offgasausgang (1c) aufweist, wobei die Membraneinheit (1) mindestens einen weiteren Permeatausgang (1c') aufweist und der Permeatausgang (1c') der Membraneinheit (1) ansaugseitig mit dem Verdichter (3) bzw. der in den Verdichter führenden Gaszufuhr leitungsmäßig verbunden ist, und wobei am oder nach dem Retentatausgang (1b) eine Druckregeleinrichtung (2) vorgesehen ist, die Verwendung einer derartigen Vorrichtung sowie ein entsprechendes Verfahren.

## Beschreibung

Die vorliegende Erfindung betrifft eine Vorrichtung und ein Verfahren zur Auftrennung eines Gasgemisches in Produktgas und Offgas mittels Gaspermeation.

Bei Gaspermeationsmembraneinheiten erfolgt die Trennung von Produktgas und Offgas mittels Permeation, wobei ein beispielsweise an Produktgas angereichertes Retentat und ein an Offgas angereichertes Permeat gewonnen werden können. Die Konzentrationen an Produktgas im Retentat und Offgas im Permeat sind unter anderem von den jeweils angewendeten Prozess-Parametern abhängig, generell ist für eine erhöhte Produktgas-Qualität immer ein erhöhter Energieeinsatz erforderlich (wegen höherem Druck, niedrigerer Ausbeute in Bezug auf eingesetztes Feedgas, etc.). Verbesserte Verfahren zur Steigerung der Produktgas-Ausbeute oder zur effizienteren Nutzung von Energie im Zuge eines derartigen Verfahrens sind daher wünschenswert. Auch ist es wünschenswert, die bei der Errichtung einer Gaspermeationsanlage anfallenden Investitionskosten möglichst gering zu halten.

Bisher sind Vorrichtungen zur Auftrennung eines Gasgemisches in Produktgas und Offgas mittels Gaspermeation derart aufgebaut, dass das druckbeaufschlagte Feedgas in einer Membraneinheit in das Retentat und in das Permeat aufgetrennt werden, wobei beispielsweise das Retentat das Produktgas und das Permeat das Offgas enthält. Nachteil dieser einstufigen Lösung sind niedrige Produktgasqualität und niedrige Produktgasausbeute, die mit einem erhöhten Energiebedarf verbunden ist. Weiters ist diese Vorrichtung nur für sehr selektive Membranen wirtschaftlich einsetzbar.

Verbesserte Vorrichtungen zur Auftrennung eines Gasgemisches in Produktgas und Offgas mittels Gaspermeation sind derart aufgebaut, dass das Permeat einer ersten Membraneinheit druckbeaufschlagt als Feedgas für eine zweite Membraneinheit verwendet wird, wobei die Retentatströme der beiden Membraneinheiten das Produktgas und der Permeatstrom der zweiten Membraneinheit das Offgas enthalten. Der Anlage kann gegebenenfalls noch ein Verdichter vorgeschaltet werden, wenn das Feedgas nicht druckbeaufschlagt vorhanden ist. Der Vorteil dieser Vorrichtung ist eine verbesserte Produktgasausbeute. Nachteil dieser Lösung sind die weiterhin niedrige Produktgasqualität und ein aufgrund der erforderlichen Verdichtung des Gases für die zweite Membraneinheit erhöhter Energiebedarf. Weiters ist diese Vorrichtung nur für sehr selektive Membranen wirtschaftlich einsetzbar.

Weiters sind Vorrichtungen bekannt, wobei das Retentat einer ersten Membraneinheit als Feedgas einer zweiten Membraneinheit verwendet wird, das Permeat der zweiten Membraneinheit dem druckbeaufschlagten Feedgas der ersten Membraneinheit beigemischt wird, das Retentat der zweiten Membraneinheit als Produktgas und das Permeat der ersten Membraneinheit als Offgas abgezogen wird. Da hier das Permeat der zweiten Membraneinheit sozusagen im Kreislauf geführt wird, muss die Dimension der Anlage und aller nötigen Teile (Kompressoren, Leitungen, Membraneinheiten, Kälteabscheider, Schwefelfeinabscheider etc.) entsprechend dem Volumsstrom des im Kreislauf geführten Permeats der ersten Membraneinheit vergrößert werden. Bei einem angenommenen Volumsstrom an Feedgas von 100 m³/h und Beimischung von 80 m³/h an Permeat der zweiten Membraneinheit zu diesem Feedgas ergibt sich vor dem Kompressor ein Gesamtvolumsstrom von 180 m³/h, gemäß welchem die Anlage zu dimensionieren ist. Vorteil dieses Verfahren ist, dass eine höhere Ausbeute an Produktgas erzielt werden kann, auch können aufgrund der zweistufigen Ausführung weniger selektive Membranen eingesetzt werden, nachteilig ist dabei die um den Faktor 1,2 bis 2,5 nötige übergroße Auslegung der Anlage und ein aufgrund der Rückführung erhöhter Energiebedarf.

Die US 4,130,403 A (D1) offenbart die Rückkopplung des Retentatausgangs einer Membraneinheit an die Gaszufuhr einer weiteren Membraneinheit, wobei dieser lediglich eine einzige Membraneinheit vorgeschaltet ist.

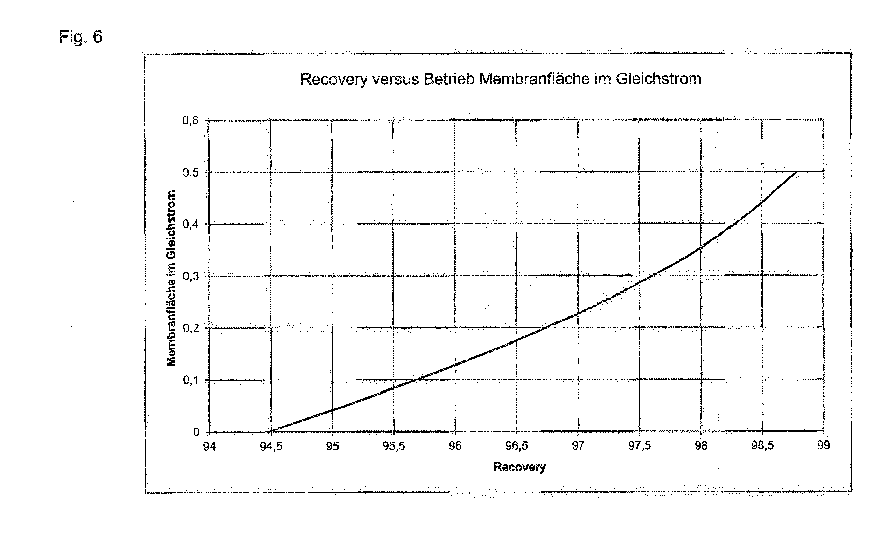

Agrawal R et al. (Journal of Membran Science, Elsevier Scientific Publ. Company, Bd. 112, Nr. 2) ist auf kaskadierte Anordnungen mit zwei Kompressoren gerichtet. Fig. 6 zeigt Rückkopplungen an den Gaseingang der jeweils ersten Membraneinheit.

Die FR 2 917 305 A1 (D3) offenbart eine Matrixanordnung einer Vielzahl von Membraneinheiten.

Aus der WO 2010/141963 A1 ist eine Vorrichtung zur Auftrennung eines Gasgemisches in Produktgas und Offgas mittels Gaspermeation mit mindestens zwei Membraneinheiten (1) und (2) und einem der ersten Membraneinheit (1) vorgeschalteten Verdichter (3) bekannt, welche Membraneinheiten (1) und (2) einen Gaseingang (1a, 2a), einen Retentatausgang (1b, 2b) und einen Permeatausgang (1c, 2c) aufweisen, wobei der Retentatausgang (1b) der ersten Membraneinheit (1) mit dem Gaseingang (2a) der zweiten Membraneinheit (2), der Permeatausgang (2c) der zweiten Membraneinheit (2) ansaugseitig mit dem Verdichter (3) bzw. der in den Verdichter führenden Gaszufuhr und der Verdichter (3) mit dem Gaseingang (1a) der ersten Membraneinheit (1) leitungsmäßig verbunden ist, Produktgas über Retentatausgang (2b) und Offgas über Permeatausgang (1c) erhalten wird.

Bei einer solchen Vorrichtung ist gemäß der WO 2010/141963 A1 vorgesehen, dass der Permeatausgang (4c) einer vorgeschalteten Membraneinheit (4) mit der Gaszufuhr des Verdichters (3) leitungsmäßig verbunden ist, wobei der Membraneinheit (4) noch mindestens eine weitere Membraneinheit (5) durch leitungsmäßige Verbindung des Retentatausgangs (5b) der weiteren Membraneinheit (5) mit dem Gaseingang (4a) der Membraneinheit (4) vorgeschaltet ist und zusätzliches Produktgas über Retentatausgang (4b) und zusätzliches Offgas über Permeatausgang (5c) erhalten wird.

All diesen Vorrichtungen gemeinsam ist, dass die einzelnen Membraneinheiten immer im Gegenstrom betrieben werden.

Aus der WO 2010/141963 A1 sind Membraneinheiten bekannt, die einen Gaseingang, einen Retentatausgang und zwei Permeatausgänge aufweisen, wobei die Permeat Räume solcher Membraneinheiten jeweils im Bereich zwischen den beiden Permeatausgängen durch eine Wand abgetrennt sein können.

Die Aufgabe der vorliegenden Erfindung besteht nun darin, eine Vorrichtung und ein Verfahren zur Auftrennung eines Gasgemisches in Produktgas und Offgas mittels Gaspermeation zur Verfügung zu stellen, welche eine geringere, durch Steuerung der Vorrichtung einstellbare Konzentration von Produktgas im Offgas sowie einen effizienteren Energieeinsatz ermöglicht.

Die Erfindung geht dabei von einer Vorrichtung zur Auftrennung eines Gasgemisches in Produktgas und Offgas mittels Gaspermeation mit einer Membraneinheit (1) und einem der Membraneinheit (1) vorgeschalteten, vorzugsweise drehzahlregelbaren Verdichter (3) aus, welche Membraneinheit (1) einen Gaseingang (1a), einen Retentat- bzw. Produktgasausgang (1b) und einen Permeat- bzw. Offgasausgang (1c) aufweist, wobei die Membraneinheit (1) mindestens einen weiteren Permeatausgang (1c') aufweist und der Permeatausgang (1c') der Membraneinheit (1) ansaugseitig mit dem Verdichter (3) bzw. der in den Verdichter führenden Gaszufuhr leitungsmäßig verbunden ist. Dabei ist erfindungsgemäß vorgesehen, dass am oder nach dem Retentatausgang (1b) eine Druckregeleinrichtung (2) vorgesehen ist. Erst durch diese Druckregeleinrichtung, welche für das Retentat einen Widerstand vorsieht und beispielsweise durch ein Ventil oder Stellventil, die Ansaugseite eines vorzugsweise drehzahlregelbaren Verdichters oder (ebenfalls vorzugsweise drehzahlregelbaren) Kompressors oder eine Zuleitung in eine Stufe eines vorzugsweise drehzahlregelbaren mehrstufigen Verdichters oder Kompressors dargestellt sein kann, ist es möglich, die erfindungsgemäße Membraneinheit retentatseitig unter Druck zu halten, indem die Druckregeleinrichtung den Vorgaben entsprechend mehr oder weniger geschlossen wird bzw. der Widerstand durch die Drehzahl des Verdichters oder Kompressors gegeben ist oder durch Regelung bzw. Steuerung der Drehzahl des Verdichters oder Kompressors eingestellt wird. Dadurch kann die Membraneinheit im Bereich des zweiten, vom Gaseingang stromabwärts gelegenen Permeatausgang (1c') zumindest teilweise kontrolliert im Gleichstrom betrieben werden, während die Membraneinheit im Bereich des ersten Permeatausgangs, wie üblich, vorwiegend im Gegenstrom betrieben wird. Daraus ergeben sich überraschenderweise wesentliche Verbesserungen beim Betrieb der erfindungsgemäßen Vorrichtung, hauptsächlich hinsichtlich einer Verringerung der Konzentration an Produktgas im Offgas aber auch hinsichtlich einer Erhöhung der Menge an Produktgas. So kann beispielsweise bei der Trennung eines hauptsächlich aus CH₄/CO₂ bestehenden Gasgemisches, beispielsweise Schwachgas, Grubengas oder Biogas, in CH₄ als Produktgas und hauptsächlich CO₂ als Offgas mittels der erfindungsgemäßen Vorrichtung eine überraschend hohe Recovery an CH₄ im Produktgas von mehr als 98%, vorzugsweise bis zu 99,8 % erreicht werden, zumindest wenn die Methankonzentration im Feedgas nicht über 30 Vol.-% liegt. Selbstverständlich kann, beispielsweise bei Vorsehen von Sensoren an geeigneter Stelle in der erfindungsgemäßen Vorrichtung, die Druckregeleinrichtung auch automatisiert betrieben werden, indem die Druckregeleinrichtung mittels einer Steuereinrichtung zur Einhaltung eines vorgegebenen, an einem oder mehreren Stellen innerhalb oder außerhalb der erfindungsgemäßen Vorrichtung gemessenen Grenzwertes geöffnet bzw. geschlossen wird. Der Grenzwert kann beispielsweise die Methankonzentration im Offgas sein, wobei in dem Fall der Sensor ein an oder nahe beim Permeatausgang (1c) vorgesehener Gassensor sein kann. Beispielsweise ist für die Freisetzung von Methan (Produktgas bzw. Retentat bei der Trennung von Biogas mittels Gaspermeation) in Deutschland die maximal emittierbare Methanmenge im Abgas auf 0,2 Vol.-% der im Biogasprozess produzierten Methanmasse begrenzt, wobei das Einhalten dieses Grenzwerts beispielsweise durch die erfindungsgemäße Vorrichtung bei Vorsehen einer Steuereinrichtung auch automatisiert vorgesehen werden kann. Dadurch, dass der Permeatausgang (1c') der Membraneinheit (1) ansaugseitig mit dem Verdichter (3) bzw. der in den Verdichter führenden Gaszufuhr leitungsmäßig verbunden ist wird vorgesehen, dass das Permeat dem Feedgas vor oder während seiner Druckbeaufschlagung zugeführt werden kann. Sofern die Zufuhr ein höheres Druckniveau erfordert, kann dieses durch gegebenenfalls zusätzlich vorgesehene Verdichter oder Kompressoren (auf welche im Folgenden nicht ausdrücklich Bezug genommen wird) bewerkstelligt werden.

Gemäß einer bevorzugten Ausführungsform der vorliegenden Erfindung ist vorgesehen, dass am oder nach dem ersten Permeatausgang (1c) ein vorzugsweise drehzahlregelbarer Verdichter (4) und/oder eine Druckregeleinrichtung (5) vorgesehen ist bzw. sind. Durch das Vorsehen einer Druckregeleinrichtung, beispielsweise eines Ventils oder Stellventils, kann bei Verringerung des Durchflusses durch das Ventil im Permeatraum der erfindungsgemäßen Vorrichtung ein Rückstau erzeugt werden, wodurch sich die im Gleichstrom betriebene Fläche der Trennmembran vergrößert. Wird andererseits am oder nach dem ersten Permeatausgang (1c) ein vorzugsweise drehzahlregelbarer Verdichter betrieben, erhöht sich der Abzug von Offgas aus dem ersten Permeatausgang (1c) und die im Gleichstrom betriebene Fläche der Trennmembran verringert sich. Beim Vorsehen von sowohl Druckregeleinrichtung als auch vorzugsweise drehzahlregelbaren Verdichter kann die erfindungsgemäße Vorrichtung somit in jede Richtung gesteuert werden.

Günstig ist auch, wenn in der Leitung vom Permeatausgang (1c') der Membraneinheit (1) ansaugseitig zum Verdichter (3) bzw. der in den Verdichter führenden Gaszufuhr eine Druckregeleinrichtung (6) vorgesehen ist. Ähnlich wie bei der zuvor beschriebenen bevorzugten Ausführungsform kann durch das Vorsehen eine Druckregeleinrichtung in der Leitung vom Permeatausgang (1c') der Membraneinheit (1) ansaugseitig zum Verdichter (3) bzw. der in den Verdichter führenden Gaszufuhr der Rückstau in den Permeatraum der erfindungsgemäßen Vorrichtung vergrößert werden (wenn die Druckregeleinrichtung, beispielsweise ein Ventil oder Stellventil, den Gasdurchfluss verringert), wodurch sich die im Gleichstrom betriebene Fläche der Trennmembran verringert. Wenn andererseits die Druckregeleinrichtung geöffnet wird, ergibt sich aus der Ansaugleistung des Verdichters (3) im Permeatraum zumindest im Bereich des Permeatausgangs (1c') ein Druckverlust, wodurch sich die im Gleichstrom betriebene Fläche der Trennmembran vergrößert. Unter den Schutzbereich der vorliegenden Erfindung fällt jedenfalls auch eine Ausführungsform, bei welcher die Leitung vom Permeatausgang (1c') der Membraneinheit (1) ansaugseitig in einen Verdichter bzw. der in den Verdichter führenden Gaszufuhr einer parallelen Membraneinheit führt. Dabei ist in der Leitung vorzugsweise ebenfalls die Druckregeleinrichtung (6) vorgesehen, welche in Abhängigkeit von der Konzentration von Produktgas im Offgas gesteuert bzw. geregelt wird.

Erfindungsgemäß wird auch vorgesehen, dass der drehzahlregelbare Verdichter (3) und die Druckregeleinrichtung (2) mit einer Steuereinrichtung (7) verbunden sind. Bei Vorsehen von beispielsweise einem Gassensor im Bereich des Permeatausgangs (1c), welcher Gassensor beispielsweise die Konzentration an Produktgas im Offgas direkt oder indirekt messen kann, ist es wie bereits zuvor erwähnt möglich, die erfindungsgemäße Vorrichtung derart zu betreiben, dass ein vorgegebener Produktgasanteil im Offgas nicht überschritten wird.

Eine weitere bevorzugte Ausführungsform der vorliegenden Erfindung ist dadurch gekennzeichnet, dass der vorzugsweise drehzahlregelbare Verdichter (4) und/oder die Druckregeleinrichtung (5) mit einer bzw. der Steuereinrichtung (7) verbunden sind. Bei Vorsehen von beispielsweise einem Gassensor im Bereich des Permeatausgangs (1c), welcher Gassensor beispielsweise die Konzentration an Produktgas im Offgas direkt oder indirekt messen kann, ist es wie bereits zuvor erwähnt möglich, die erfindungsgemäße Vorrichtung derart zu betreiben, dass ein vorgegebener Produktgasanteil im Offgas nicht überschritten wird.

Dabei ist ebenfalls günstig, wenn die Druckregeleinrichtung (6) mit einer bzw. der Steuereinrichtung (7) verbunden ist. Bei Vorsehen von beispielsweise einem Gassensor im Bereich des Permeatausgangs (1c), welcher Gassensor beispielsweise die Konzentration an Produktgas im Offgas direkt oder indirekt messen kann, ist es wie bereits zuvor erwähnt möglich, die erfindungsgemäße Vorrichtung derart zu betreiben, dass ein vorgegebener Produktgasanteil im Offgas nicht überschritten wird.

Gemäß einem weiteren Aspekt der vorliegenden Erfindung wird die genannte Vorrichtung zur Trennung eines hauptsächlich aus CH₄/CO₂ bestehenden Gasgemisches in CH₄ als Produktgas und hauptsächlich CO₂ als Offgas eingesetzt. Beispiele für derartige Gasgemisches sind Schwachgas, Grubengas, Biogas et cetera.

Noch ein weiterer Aspekt der vorliegenden Erfindung betrifft ein Verfahren zur Auftrennung eines Gasgemisches in Produktgas und Offgas mittels Gaspermeation, dadurch gekennzeichnet, dass das Permeat einer Membraneinheit (1) teilweise im Gleichstrom und teilweise im Gegenstrom gewonnen wird, wobei das im Gleichstrom gewonnene Permeat druckbeaufschlagt und der Membraneinheit (1) wieder als Feedgas zugeführt wird, das die Gegenstrom gewonnene Permeat als Offgas abgezogen wird, und weiters das Retentat der Membraneinheit (1) als Produktgas abgezogen wird, wobei der Abzug des im Gleichstrom gewonnenen Permeats in Abhängigkeit von der Konzentration von Produktgas im Offgas gesteuert bzw. geregelt wird. Der Abzug des im Gleichstrom gewonnenen Permeats erfolgt dabei über die genannte Druckbeaufschlagung, wodurch das Permeat der Membraneinheit wieder als Feedgas zugeführt wird.

Vorzugsweise ist dabei vorgesehen, dass der Druck des Retentats in der Membraneinheit (1) erhöht wird. Wie bereits ausgeführt wird beispielsweise durch Vorsehen eines Widerstandes, wie oben hinsichtlich des Widerstandes (2) geoffenbart, der Druck des Retentat in der Membraneinheit erhöht, was in weiterer Folge zu einer Vermehrung des im Gleichstrom gewonnenen Permeats führt.

Die vorliegende Erfindung wird nun unter Bezugnahme auf die beiliegenden Figuren näher erklärt. Dabei zeigen:
Fig. 1 ein Blockschaltbild einer erfindungsgemäßen Vorrichtung mit einer Membraneinheit (1), einer Druckregeleinrichtung (2) und einem der Membraneinheit (1) vorgeschalteten Kompressor bzw. Verdichter (3);
Fig. 2 ein Blockschaltbild der Vorrichtung von Fig. 1 mit zusätzlich einer zweiten Druckregeleinrichtung (5) sowie einem zweiten Kompressor bzw. Verdichter (4);
Fig. 3 ein Blockschaltbild der Vorrichtung von Fig. 2 mit zusätzlich einer dritten Druckregeleinrichtung (6) sowie
Fig. 4 ein Blockschaltbild der Vorrichtung von Fig. 3 mit zusätzlich einer Steuereinrichtung (7),
Fig. 5 einen Vergleich zwischen den erzielten Werten bei Betrieb desselben Membraneinheit im Gegenstrom, im Gleichstrom sowie gemäß der vorliegenden Erfindung (mit teilweisem Betrieb im Gleichstrom) und
Fig. 6 die hinsichtlich Recovery erzielten Ergebnisse bei stufenloser Veränderung des Anteils an im Gleichstrom betriebene Membranfläche, wobei dieselben Membraneinheit wie für die Ergebnisse von Figur 5 verwendet wurde.

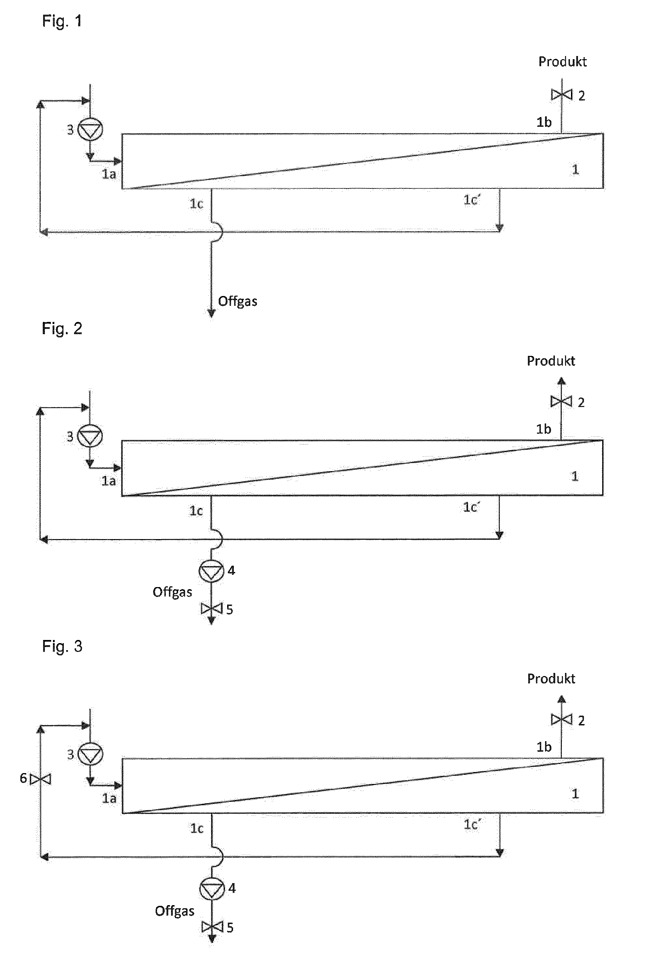

Die Membraneinheit gemäß Fig. 1 weist einen Gaseingang (1a), einen Retentatausgang (1b) und einen zusätzlichen Permeatausgang (1c') auf. In der Membraneinheit (1) wird im Gegenstrombereich anfallendes Offgas über der Permeatausgang (1c) abgeführt, im Gleichstrombereich über Permeatausgang (1c') und mittels des Verdichters (3) abgesaugtes Permeat wird über den Verdichter (3) der Membraneinheit (1) wieder als Feedgas wieder zugeführt. Dadurch wird über Verdichter (3), Gaseingang (1a) und Permeatausgang (1c') in der Membraneinheit (1) im Gleichstrombereich abgetrenntes Offgas im Kreislauf geführt. Produktgas wird über den Retentatausgang (1b) abgeführt, wobei die dort vorgesehene Druckregeleinrichtung (2) dazu dient, den Druck im Retentatraum der Membraneinheit (1) zu erhöhen, wodurch der erfindungsgemäße Betrieb der Trennmembran im teilweisen Gleichstrom erst kontrollierbar wird.

In Fig. 2 wird die Ausführungsform von Fig. 1 gezeigt, wobei in der an den Permeatausgang (1c) anschließenden Leitung eine zweiten Druckregeleinrichtung (5) sowie ein zweiter, vorzugsweise drehzahlregelbarer Kompressor bzw. Verdichter (4) vorgesehen ist. Bei Schließen der Druckregeleinrichtung (5) staut sich Offgas zurück in den Retentatraum der Membraneinheit (1), wodurch zwangsweise mehr Retentat beim Retentatausgang (1c') austreten muss, wodurch sich die im Gleichstrom betriebene Fläche der Trennmembran vergrößert. Wird andererseits die Druckregeleinrichtung (5) vollständig geöffnet und der vorzugsweise drehzahlregelbare Verdichter (4) gestartet, muss zwangsweise mehr Retentat beim Retentatausgang (1c) austreten, wodurch sich der Abzug von Offgas aus dem ersten Permeatausgang (1c) erhöht und sich die im Gleichstrom betriebene Fläche der Trennmembran verringert.

In Fig. 3 wird die Ausführungsform von Fig. 2 gezeigt, wobei in der Leitung zwischen Permeatausgang (1c') und Verdichter (3) bzw. Zugangsleitung zum Verdichter (3) eine weitere Druckregeleinrichtung (6) vorgesehen ist. Mittels dieser Druckregeleinrichtung (6) kann der Austritt von Permeat aus dem Permeatraum der Membraneinheit (1) über den Permeatausgang (1c') unabhängig von der Leistung des Verdichters (3) verringert werden, wodurch sich die Druckverhältnisse im Permeatraum nahe dem Permeatausgang (1c') verändern. Bei erzeugtem Rückstau drängt das Retentat vermehrt zum Retentatausgang (1c), wodurch sich die im Gleichstrom betriebene Fläche der Trennmembran ebenfalls verringert.

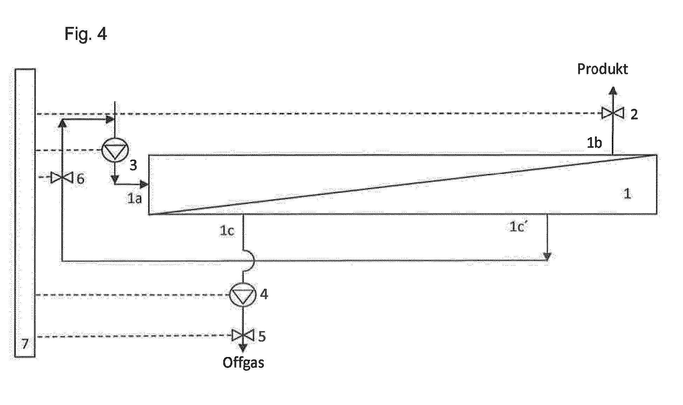

In Figur 4 wird die Ausführungsform von Fig. 3 gezeigt, wobei die Druckregeleinrichtungen (2, 5, 6) und die Verdichter (3, 4) mit einer Steuereinrichtung (7) verbunden sind. Es sei in diesem Zusammenhang ausdrücklich angemerkt, dass erfindungsgemäß auch eine Verbindung von nur einzelnen der Einrichtungen (2, 3, 4, 5, 6) mit der Steuereinrichtung vorgesehen ist. Allfällige für den Betrieb der Steuereinrichtung nötige Sensoren, beispielsweise Gassensoren, Drucksensoren und/oder Durchflusssensoren, werden in den Figuren 1 bis 4 nicht gezeigt. Die Steuereinrichtung (7) ist derart ausgelegt, dass sie das Öffnen und Schließen der Druckregeleinrichtungen (2, 5, 6) unabhängig voneinander sowie eine ebenfalls unabhängige Regelung der Drehzahl der Verdichter (3, 4) vorsehen kann.

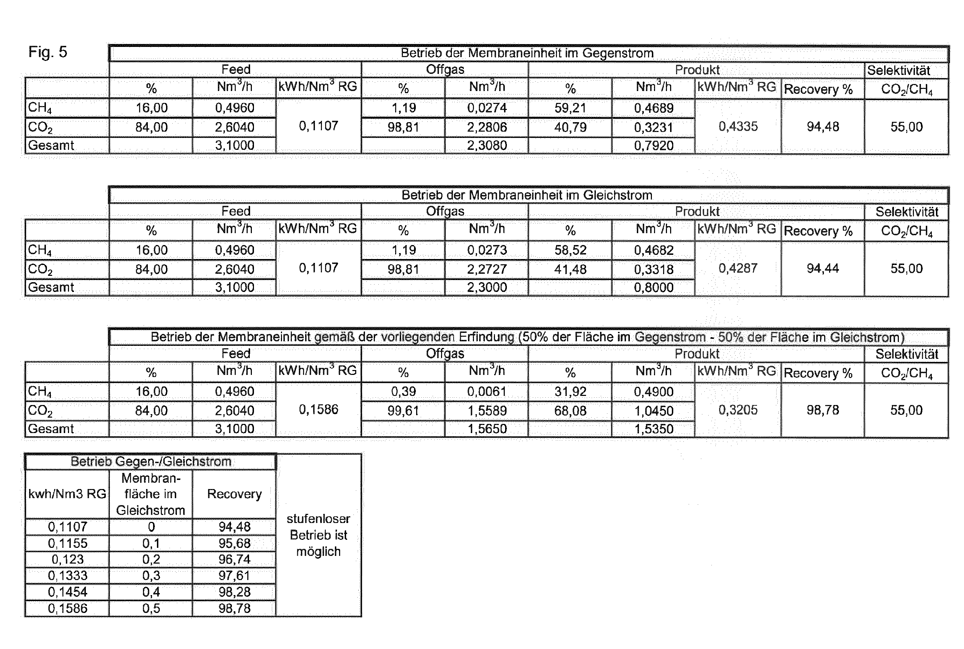

Aus Figur 5 ist ersichtlich, dass sich die Werte für den Betrieb der Membraneinheit im Gegenstrom bzw. im Gleichstrom nur wenig voneinander unterscheiden, erst bei teilweisem Betrieb der Membranfläche im gegen Strom und teilweisem Betrieb im Gleichstrom ist ein deutliches Anwachsen der Recovery erkennbar. Dies ist insofern günstig, als bei Verwendung der erfindungsgemäßen Membraneinheit bzw. Betrieb der Membraneinheit gemäß der vorliegenden Erfindung bei einer Methankonzentration im Feedgas nicht über 30 Vol.% der Methananteil im Offgas auf bisher unerreichte Werte reduziert werden kann, was insbesondere hinsichtlich der Effizienz der Abtrennung von Methan aus dem verwendeten Feedgas wesentliche Vorteile bringt. Es versteht sich, dass die erfindungsgemäße Vorrichtung nicht nur für die Abtrennung von Methan aus einem methanhältigen Feedgas einsetzbar ist sondern, bei Verwendung von für das jeweilige Gasgemisch geeigneten Permeationsmembranen, die Recovery an Produktgas für jegliches Gasgemisch wesentlich steigern kann.

## Patentansprüche

1. Vorrichtung zur Auftrennung eines Gasgemisches in Produktgas und Offgas mittels Gaspermeation mit einer Membraneinheit (1) und einem der Membraneinheit (1) vorgeschalteten, vorzugsweise drehzahlregelbaren Verdichter (3), welche Membraneinheit (1) einen Gaseingang (1a), einen Retentat- bzw. Produktgasausgang (1b) und einen Permeat- bzw. Offgasausgang (1c) aufweist, wobei die Membraneinheit (1) mindestens einen weiteren Permeatausgang (1c') aufweist und der Permeatausgang (1c') der Membraneinheit (1) ansaugseitig mit dem Verdichter (3) bzw. der in den Verdichter führenden Gaszufuhr leitungsmäßig verbunden ist, **dadurch gekennzeichnet, dass** am oder nach dem Retentatausgang (1b) eine Druckregeleinrichtung (2) vorgesehen ist.

2. Vorrichtung nach Anspruch 1, **dadurch gekennzeichnet, dass** am oder nach dem ersten Permeatausgang (1c) ein vorzugsweise drehzahlregelbarer Verdichter (4) und/oder eine Druckregeleinrichtung (5) vorgesehen ist bzw. sind.

3. Vorrichtung nach Anspruch 1 oder 2, **dadurch gekennzeichnet, dass** in der Leitung vom Permeatausgang (1c') der Membraneinheit (1) ansaugseitig zum Verdichter (3) bzw. der in den Verdichter führenden Gaszufuhr eine Druckregeleinrichtung (6) vorgesehen ist.

4. Vorrichtung nach Anspruch 1, **dadurch gekennzeichnet, dass** der drehzahlregelbare Verdichter (3) und die Druckregeleinrichtung (2) mit einer Steuereinrichtung (7) verbunden sind.

5. Vorrichtung nach Anspruch 2 oder 4, **dadurch gekennzeichnet, dass** der vorzugsweise drehzahlregelbare Verdichter (4) und/oder die Druckregeleinrichtung (5) mit einer bzw. der Steuereinrichtung (7) verbunden sind.

6. Vorrichtung nach Anspruch 3 oder 5, **dadurch gekennzeichnet, dass** die Druckregeleinrichtung (6) mit einer bzw. der Steuereinrichtung (7) verbunden ist.

7. Verwendung einer Vorrichtung nach einem der Ansprüche 1 bis 6 zur Trennung eines hauptsächlich aus CH₄/CO₂ bestehenden Gasgemisches in CH₄ als Produktgas und CO₂ als Offgas.

8. Verfahren zur Auftrennung eines Gasgemisches in Produktgas und Offgas mittels Gaspermeation, **dadurch gekennzeichnet, dass** das Permeat einer Membraneinheit (1) teilweise im Gleichstrom und teilweise im Gegenstrom gewonnen wird, wobei das im Gleichstrom gewonnene Permeat druckbeaufschlagt und der Membraneinheit (1) wieder als Feedgas zugeführt wird, das im Gegenstrom gewonnene Permeat als Offgas abgezogen wird, und weiters das Retentat der Membraneinheit (1) als Produktgas abgezogen wird, wobei der Abzug des im Gleichstrom gewonnenen Permeats in Abhängigkeit von der Konzentration von Produktgas im Offgas gesteuert bzw. geregelt wird.

9. Verfahren nach Anspruch 8, **dadurch gekennzeichnet, dass** der Druck des Retentats in der Membraneinheit (1) erhöht wird.
